(19) Europäisches Patentamt
European Patent Office
Office européen des brevets

(11) Veröffentlichungsnummer: **0 018 002**
**B1**

# (12) EUROPÄISCHE PATENTSCHRIFT

(45) Veröffentlichungstag der Patentschrift: **09.02.83**

(51) Int. Cl.³: **C 12 Q 1/00**, G 01 N 27/48, C 12 Q 1/56

(21) Anmeldenummer: **80102107.2**

(22) Anmeldetag: **18.04.80**

## (54) Neues Bestimmungsverfahren für Proteasen und Antiproteasen.

(30) Priorität: **24.04.79 FR 7910305**
**17.12.79 FR 7930820**

(43) Veröffentlichungstag der Anmeldung:
**29.10.80 Patentblatt 80/22**

(45) Bekanntmachung des Hinweises auf die Patenterteilung:
**09.02.83 Patentblatt 83/6**

(84) Benannte Vertragsstaaten:
**CH DE FR GB IT LI NL**

(56) Entgegenhaltungen:
**EP-A-0 004 256**
**FR-A-2 315 695**
**FR-A-2 317 280**
**US-A-3 367 849**
**ANALYTICAL CHEMISTRY, Band 45, Nr. 6, Mai 1973, Seiten 1006–1008; Columbus, Ohio, USA, J. LINDQUIST: »Carbon Paste Electrode with a Wide Anodic Potential Range.«**

(73) Patentinhaber: **Jozefonvicz, Marcel, 65, Deuxième Avenue, Le Lys F-60260 Lamorlaye (FR)**
Patentinhaber: **Nigretto, Jean Maxime, 349, Parc De Cassan, F-95290 L'Isle Adam (FR)**

(72) Erfinder: **Jozefonvicz, Marcel, 65, Deuxième Avenue, Le Lys F-60260 Lamorlaye (FR)**
Erfinder: **Nigretto, Jean Maxime, 349, Parc De Cassan, F-95290 L'Isle Adam (FR)**

(74) Vertreter: **Ores, Irène et al, CABINET ORES 6, Avenue de Messine, F-75008 - Paris (FR)**

**EP 0 018 002 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neues Bestimmungsverfahren für Proteasen und Antiproteasen

Die vorliegende Erfindung betrifft ein neues Bestimmungsverfahren für Proteasen und Antiproteasen und insgesondere Proteasen und Antiproteasen der Blutgerinnungssysteme und des Komplements.

Die Information über die Enzymaktivität des Blutes und insbesondere der Proteasen des Typs Trypsin (»trypsin-like«) sind entscheidend für die Kenntnis der eventuellen Anomalien der Blutgerinnungsfaktoren. Diese Bestimmung liefert ebenfalls sehr oft äußerst wichtige Angaben über schwerwiegendere Störungen im Organismus. Aus diesem Grunde ist es von größter Wichtigkeit, über ein sicheres und genaues Bestimmungsverfahren für diese Enzyme zu verfügen. Die derzeit benutzten Bestimmungsmethoden beruhen hauptsächlich auf einer photometrischen Bestimmung (einfache Photometrie, Spektrophotometrie bzw. Fluoreszenzphotometrie) der nach der Spaltung spezifischer Substrate durch die zu bestimmenden Enzyme erhaltenen chromogenen bzw. fluoreszierenden Gruppen.

Die meisten der benutzten Substrate sind synthetische Substrate. Sie sind für die Bestimmung von Blutenzymen ebenso wie die Untersuchung der Reaktionen, welche die Bildung, die Inhibition bzw. den Verbrauch dieser Enzyme hervorrufen, und sogar für die Bestimmung von Faktoren, die diese verschiedenen Reaktionen beeinflussen oder die daran teilnehmen, bestens geeignet (vergleiche insbesondere die französischen Patente Nr. 2 183 188, 2 317 278, 2 317 280, 2 293 439).

Leider sind diese eleganten Bestimmungsverfahren nur in einem homogenen Medium genau. In heterogenen Medien, wie den biologischen Flüssigkeiten (und insbesondere dem Gesamtblut) läßt ihre Genauigkeit bedeutend nach. Um diesen entscheidenden Nachteil zu umgehen, ist vorgeschlagen worden, zentrifugiertes Blut zu benutzen, das heißt Blut ohne Feststoffe, welches infolgedessen für photometrische Verfahren besser geeignet ist. Über die Tatsache hinaus, daß dieses Ausschleudern entsprechende Geräte erfordert, die schwer und kompliziert sind, und daß es eine Quelle unvermeidlicher Verluste bildet, ergibt sich daraus noch ein gewisser »Traumatismus« des Blutes, der zur Freigabe nicht vernachlässigbarer Mengen von verschiedenen Faktoren aus den Blutplättchen führt, wobei diese Faktoren selbst eine gewisse Enzymaktivität besitzen und infolgedessen die Ergebnisse der Bestimmung verfälschen.

Ziel der vorliegenden Erfindung ist infolgedessen ein neues Bestimmungsverfahren der vorgenannten Enzyme zu schaffen, das der Praxis besser angepaßt ist als die bisher bekannten Bestimmungsverfahren, und dies insbesondere durch die Tatsache, daß es einfach, genau und zuverlässig ist, und daß es in homogenen oder heterogenen Medien ebenso wie in Lösungen oder Suspensionen angewandt werden kann.

Gegenstand der vorliegenden Erfindung ist ein neues Bestimmungsverfahren für Proteasen und Antiproteasen, und insbesondere Proteasen und Antiproteasen der Blutgerinnungssysteme und des Komplements, dadurch gekennzeichnet, daß man in einem wäßrigen Medium das Enzym mit einem Substrat A—B zusammenbringt, worin A ein Peptidrest, dessen terminales Stickstoffatom gegebenenfalls substituiert und dessen C-terminale Aminosäure Arginin ist, oder N-substituiertes Arginyl, und B den Rest eines Amins H—B bedeutet, das elektrochemisch oxydiert oder reduziert werden kann, und das abgespaltene Amin mittels Amperometrie bestimmt.

Aus der US-A-3 367 849 ist eine Bestimmungsmethode für Glucose bekannt, welche auf der amperometrischen Bestimmung von Ferricyanid-Ionen beruht.

Damit diese elektrochemische Bestimmung zufriedenstellend erfolgen kann, darf das spezifische Substrat des entsprechenden Enzyms nicht unter den Bedingungen der Bestimmung elektrochemisch oxydiert oder reduziert werden, es müssen leitende oder halbleitende Elektroden benutzt werden und darüber hinaus muß das Produkt der Hydrolyse des Substrats durch das Enzym selbst elektroaktiv und die gemessene Stromstärke direkt proportional der Menge an Enzymen im betreffenden Medium sein.

Gemäß einer vorteilhaften Ausführungsart des erfindungsgemäßen Bestimmungsverfahrens wird die Zunahme der Konzentration der elektroaktiven Verbindung, die das Produkt der Hydrolyse des Substrates ist, in der Meßzelle periodisch anhand der Änderung des Oxidations- oder Reduktionsstromes verfolgt.

Entsprechend einer besonderen Ausführungsform ergibt sich der genannte Oxydations- oder Reduktionsstrom aus der Höhe des Peaks in der Strom-Spannungscharakteristik, der resultiert, wenn eine Wechselspannung angelegt wird.

Die Höhe des Strom-Spannungs-Peaks ist in der Tat proportional zur Konzentration des elektroaktiven Produktes der enzymatischen Hydrolyse, das in der Lösung und auf der Oberfläche der Meßelektrode vorhanden ist.

Gemäß einer vorteilhaften Ausführungsart des erfindungsgemäßen Bestimmungsverfahrens wird die Meßelektrode vor der eigentlichen Bestimmung dadurch vorkonditioniert, daß ein Sägezahnpotential angelegt wird, deren Amplitude zwischen $-0{,}1$ V und $+0{,}4$ V schwankt und sich mit einer Geschwindigkeit von 0,2 V/sec ändert, bis die auf einem Aufnahmegerät aufgenommene Meßkurve $i = f(U)$ eine Grenzkurve erreicht hat.

Diese Vorkonditionierung ist erforderlich, weil die während der Hydrolyse gemessenen Stromwerte

sehr niedrig sind. Sie dient dazu, der Elektrode vor jedem Zyklus einen reproduzierbaren und genau definierten Oberflächenzustand zu verleihen. Dank dieser Vorkonditionierung sind die gemäß der vorliegenden Erfindung durchgeführten elektrochemischen Messungen besonders empfindlich und zuverlässig.

Entsprechend einer vorteilhaften Ausführungsart des erfindungsgemäßen Verfahrens ist die Meßelektrode eine Elektrode aus einem Edelmetall wie Platin, Gold, Silber oder auch Edelstahl oder eine Elektrode aus Kohlenstoffpaste.

Gemäß einer weiteren vorteilhaften Ausführungsart kann das Bestimmungsmedium sowohl wäßrig als auch nicht wäßrig sein und die Bestimmung im gesamten pH-Bereich erfolgen.

Es ist möglich, die Bestimmung kinetisch oder als Endpunktmethode auszuführen.

In einer bevorzugten Ausführungsform des Verfahrens besteht das Substrat aus einem Oligopeptid, an das ein aromatisches oder heterozyklisches Amin oder Polyamin gebunden ist. In dem erfindungsgemäßen Verfahren spaltet das zu bestimmende Enzym die Bindung zwischen der carboxyterminalen Aminosäure und dem Amin oder Polyamin. Das Meßverfahren besteht darin, die Menge des freigesetzten Amins oder Polyamins elektrochemisch zu bestimmen.

Geeignete Amine oder Polyamine sind:

p-Amino-Diphenylamin,
4,4′-Benzidin oder
o-Dianisidin.

Weitere verwendbare Amine sind:

p-Nitroanilin und
4-Methoxy-2-Naphthylamin.

In einer bevorzugten Ausführungsform enthält das Substrat den folgenden Aminosäure- oder Peptidrest:

Benzoyl-Arginyl,
Tosyl-Arginyl,
Tosyl-L-Glycyl-L-Prolyl-L-Arginyl,
H-D-Phenylalanyl-L-Pipecolyl-L-Arginyl,
N-Benzoyl-L-Phenylalanyl-L-Valyl-L-Arginyl oder
H-D-Phenylalanyl-L-Pipecolyl-L-Arginyl.

Entsprechend einer Besonderheit dieser Ausführungsart besteht das Substrat aus H-D-Phenylalanyl-L-Pipecolyl-L-Arginyl-p-Amino-Diphenylamid-Dihydrochlorid (H-D-Phe-Pip-Arg-PADA, 2 HCl), wobei die enzymatische Reaktion entsprechend der nachstehenden Reaktionsgleichung stattfindet:

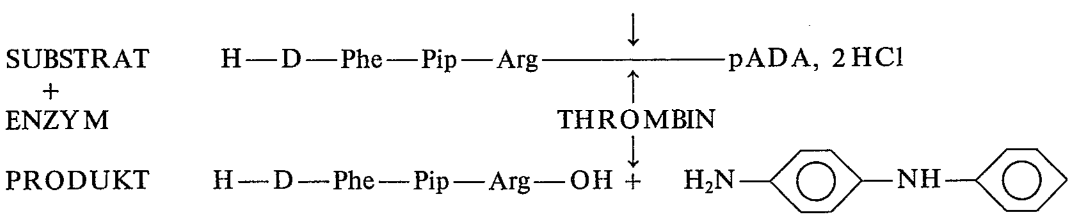


Elektroaktives Produkt: p-Amino-Diphenylamin (pADA)

Gemäß einer weiteren vorteilhaften Ausführungsart des Gegenstandes der Erfindung und um die Fällung des Substrates zu vermeiden, werden dem Reaktionsmedium 2 bis 10% Dimethylsulfoxyd zugefügt.

Die vorliegende Erfindung betrifft insbesondere das neue elektrochemische Bestimmungsverfahren für Proteasen und Antiproteasen der Blutgerinnungssysteme und des Komplements, und zwar des Trypsins, des Plasmins, des Kallikreins, des Faktors XII (Hageman-Faktor), des Faktors XI (Antezedenz-Plasma-Thromboplastin), des Faktors X (Stuart-Prower-Faktor), des Faktors IX (Christmas-Faktor), der Enzyme $C_1$ und $C_3$ des Komplementsystems, der Antiproteasen, wie das Antithrombin III, $C_1$-Inaktivator, das Alpha$_2$-Makroglobulin, das Antiplasmin, das Alpha$_2$-Antitrypsin usw., ebenso wie die zur Durchführung dieses geeignete Mittel und Verfahren, die vom Verfahren und den Substraten der vorliegenden Erfindung Gebrauch machen.

Die Erfindung läßt sich mit Hilfe der nachstehenden ergänzenden Beschreibung, die sich auf Ausführungsbeispiele des elektrochemischen Bestimmungsverfahrens für Proteasen und Antiproteasen der vorliegenden Erfindung bezieht, besser verstehen.

Es wird jedoch darauf hingewiesen, daß die nachstehenden Beispiele ebenso wie die Beispiele der

Kalibrierungskurven und der kinetischen Kurven der Hydrolyse des D,L-BAPADA, die in den zugehörigen Zeichnungen dargestellt sind, ebenso wie das Beispiel einer Bestimmung in einem nicht wäßrigen Medium und das Beispiel für die Vorbereitung einer Elektrode mit Kohlenstoffpaste lediglich zur Illustration des Gegenstandes der Erfindung gegeben werden und keineswegs als Beschränkung zu verstehen sind.

## Beispiel

### I. Benützte Geräte

a) Ein Potentiostat, der einen konstanten Potentialunterschied zwischen der Meßelektrode und der Bezugselektrode aufrechterhalten soll. Ein Sägezahnfunktionsgenerator, der ein zwischen $-0{,}1$ und $+0{,}4$ V mit einer Geschwindigkeit von 0,2 V/sec sich änderndes Potential erzeugt. Ein graphisches Registriergerät mit guter dynamischer Leistung. Eine Zelle aus Polytetrafluoräthylen mit einem Inhalt von 5 ml, die drei Elektroden aufnehmen kann.

b) Meßelektrode: Sie besteht aus einer Platinplatte, die von einem Zylinder aus Tetrafluoräthylen umgeben ist (Oberfläche 0,0314 $cm^2$).

c) Bezugselektrode: Kalomel-Elektrode (SCE), in bezug auf welche alle Spannungswerte gemessen werden.

d) Hilfselektrode: Sie dient dazu, den Durchgang des Stromes zu gewährleisten. Sie besteht lediglich aus einem beständigen metallisch leitendem Draht (Platin).

e) Lösungen: Die Stammlösungen des Enzyms (Lösung E) und des Substrats (Lösung S) werden frisch zubereitet. Die Versuchslösung, welcher zum Zeitpunkt $t = 0$ der Kinetik eine gegebene Menge von E beigefügt wird, wird A genannt. Der Puffer pH 8,15 setzt sich aus Veronal® 0,025 M und NaCl mit der Ionenstärke $F = 0{,}15$ zusammen.

f) Beispiel einer Elektrode aus Kohlenstoffpaste: Es werden 15 g reines Kohlenstoffpulver mit 9 ml Paraffinöl, beispielsweise dasjenige, das im Handel unter der Marke Nujol® verkauft wird, vermischt. Aus der einwandfrei vermischten Paste kann eine Elektrode mit einer Fläche von 0,16 $cm^2$ und einer Stärke von 3 mm geformt werden.
Die Figuren 8 und 9 der Zeichnungen zeigen eine derartige Elektrode, und zwar als Frontansicht (Figur 8) und als Ansicht von unten (Figur 9).
Die gut homogenisierte Paste wird in den Hohlraum 1 eines Zylinders 2 gedrückt, der zum Beispiel (aber nicht ausschließlich) aus Polytetrafluoräthylen hergestellt ist. Dieser Zylinder 2 ist in seiner Mitte hohl, damit ein feiner zylindrischer Stab 3 aus Kohlenstoff eingeführt werden kann, wobei dieser Stab die Fläche der Elektrode 4 mit dem elektrischen Schaltkreis (nicht auf der Zeichnung aufgeführt) verbindet. Die Fläche 4 muß unbedingt vor jeder Bestimmung erneuert werden.

### II. Vorkonditionierung der Elektrode

Die Meßelektrode wird in der Lösung A ohne Enzym vorkonditioniert, um ihr vor jedem Zyklus einen reproduzierbaren und einwandfrei definierten Oberflächenzustand zu verleihen.

Die Vorkonditionierung wird dadurch erreicht, daß ein zeitlich verändertes Potential mit Sägezahnprofil angelegt wird, wobei das Potential zwischen $-0{,}1$ und $+0{,}4$ V schwankt und sich mit einer Geschwindigkeit von 0,2 V/sec ändert.

Die Konditionierung ist beendet, wenn auf dem Aufnahmegerät das Signal $i = f(U)$ nach etwa zwei Minuten eine Grenzkurve erreicht hat.

Die Elektrode kann dann für die kinetischen Bestimmungen benutzt werden, wozu alle 60 Sekunden dasselbe Sägezahnpotential wie bei der Konditionierung angelegt wird.

### III. Aufnahme des Grundstromes

Nach der Konditionierung zeigt die Aufnahme der beiden ersten Zyklen (mit einem Abstand von 60 Sekunden) das Profil des Untergrundstromes, dessen Werte von den Stromwerten abgezogen werden müssen, die während der Hydrolyse gemessen werden.

Figur 1 zeigt den zeitlichen Verlauf der angelegten Sägezahnspannung, die vor und nach der Zugabe der Enzymlösung (2) angelegt wird. Alle 60 Sekunden wird ein einzelner Sägezahnimpuls ausgelöst. Der typische Untergrundstrom (1) wird während eines weiteren Impulses aufgezeichnet.

Wenn ein mit einer Genauigkeit von 0,01 Mikroampere reproduzierbarer Untergrundstrom (1) erreicht worden ist, wird das Enzym zum Zeitpunkt $t = 0$ der Auslösung eines weiteren Impulses zugegeben.

Dieses Signal wird nicht aufgenommen. Die Aufnahme der darauffolgenden Signale erlaubt es, alle 60 Sekunden den Fortschritt der Hydrolyse des Substrates durch das Enzym zu verfolgen.

## IV. Graphische Auswertung der Ergebnisse

Die beim Höchstwert einer jeden Welle $i = f(U)$ aufgenommenen Stromwerte sind, nach Abzug des Untergrundstromes bei dem betreffenden Potential, proportional zur Konzentration der während der Hydrolyse freigesetzten elektroaktiven Spezies. Die Stromwerte ändern sich linear mit der Zeit im Falle kurzer Hydrolysezeiten. Der Zusammenhang der Enzymkonzentration E (in µg/ml) mit der Hydrolysegeschwindigkeit zur Zeit 0 (durch das Verhältnis $(di/dt)_0$ in µA/min dargestellt, das heißt durch die Steilheit der Kurve $i = f(t)$ am Ursprung) für eine Elektrode mit einer Fläche von I $cm^2$ wird durch die nachstehende Formel dargestellt:

$$E = K(di/dt)_0$$

Der Koeffizient K hängt von der Art des Enzyms und des Substrates, die benutzt werden, ab.

## V. Aufzeichnung der zyklischen Voltammogramme des pADA mit einer Kohlenstoffpasten-Elektrode

Figur 2 stellt die mit pH 8,15 und einer Abtastgeschwindigkeit von 0,2 V/sec erhaltenen Voltammogramme dar. Die Elektrode ist eine Elektrode mit Kohlenstoffpaste. Der Oxydationsscheitelwert des pADA — 0,22 mM — (Kurve a) ist das Ergebnis einer Übertragung zweier Elektronen in einer Potentialgegend, wo der Grundstrom durch die Sauerstoffreduktion nicht beeinflußt wird.

Die in Figur 2 dargestellte Kurve b zeigt das elektroaktive Verhalten des Substrats im Bereich anodischer Potentialwerte an, und zwar in diesem Falle des D,L-BAPADA in einer Konzentration von vl/mM. Wie einwandfrei aus Figur 2 ersichtlich, ist das BAPADA zugeordnete Maximum um −0,3 gegen die Oxydationsspitze von pADA verschoben. Dies ist wahrscheinlich auf die Benzoyl-Arginylgruppe zurückzuführen. Andererseits kann die Tatsache, daß die Oxydationsspitze beim Substrat wesentlich niedriger liegt, dadurch erklärt werden, daß der Diffusionskoeffizient des Substrats ungefähr 40mal kleiner ist als derjenige des freigegebenen Amins. Die Kurve c stellt die Strom-Spannungs-Charakteristik dar, die sich dann ergibt, wenn das Substrat (D,L-BAPADA, 1/mM) und das Hydrolyseprodukt (pADA 0,22 mM) zusammen vorhanden sind: ungeachtet der anodischen Verschiebung der Spitze im Falle von pADA gleichen die Voltammogramme a und b bezüglich der Stromwerte der Kurve c. Dieses amperometrische Verfahren zeigt, daß die lineare Beziehung zwischen der Maximal-Amplitude und der Konzentration des Amins (pADA) das Ergebnis einer ausreichenden Trennung der anodischen Strommaxima ist.

## VI. Aufzeichnung der Amplitude der anodischen Maxima als Funktion der pADA-Konzentration an einer Platinelektrode

Figur 3 stellt die voltametrische Kalibrierungskurve und die anodischen Strommaxima (als Ordinate) in Abhängigkeit von der pADA-Konzentration dar.

Die mit × dargestellten Meßpunkte gelten für pADA allein,
die Meßpunkte ○ für PADA in Anwesenheit von 1 mM D,L-BAPADA und
die Punkte △ für PADA in Anwesenheit von 75 µg/ml Trypsin.

Die Bedingungen waren die folgenden:

| | |
|---|---|
| Temperatur | 30° C |
| pH | 8,15 (TRIS-Puffer) |
| hinzugefügte DMSO-Menge | 6,35% |
| Abtastgeschwindigkeit | 0,2 V/sec |
| polierte Platinelektrode. | |

Wie aus Figur 3 ersichtlich, ist die Kurve eine perfekte Gerade. Lediglich ab 100 µg/ml Trypsin verändert sich das Profil des Voltammogrammes des pADA leicht, aber es zeigt sich keine Ladungsübertragung für die zwischen −0,1 und +0,4 V (Kalomel-Elektrode-ECS) liegenden Dreieckfunktion bei der Abtastgeschwindigkeit von ca. 0,2 V/sec.

Obwohl die Zugabe der Blutgerinnungsfaktoren des Blutes wie Fibrinogen, Prothrombin oder Thrombin zu ihrer Adsorption auf der Platinelektrode führt (vergleiche insbesondere L. Duic et al., J. Electrochem. Soc. 120 [1973], Seiten 348—361), und obwohl bei diesen Enzymen Ladungsübertragungsprozesse stattfinden, die zum Auftreten von zwei Maxima (einem anodischen und einem kathodischen) führen, können diese Maxima erst nach einer mindestens 10 Minuten dauernden zyklischen Abtastung von 0,05 V/sec geortet werden, was unter den Bedingungen der vorliegenden Erfindung nie der Fall ist,

und dies um so mehr, als die in der vorliegenden Erfindung geforderten Bedingungen, wie das Vorhandensein des Puffers (pH 8,15) und die Vorkonditionierung der Elektrode, das Auftreten dieser beiden Maxima praktisch unmöglich machen.

VII. Messung der Enzymaktivität von Trypsin

Figur 4 stellt die während der Hydrolyse von D,L-BAPADA mit Trypsin aufgenommenen Voltammogramme der Oxidation des PADA dar.

Bedingungen:

| | |
|---|---|
| Temperatur | 30° C |
| pH | 8,15 (TRIS-Puffer) |
| DMSO | 6,35% |
| polierte Platinelektrode | |
| Abtastgeschwindigkeit | 0,2 V/sec |

Die Enzymlösung wird am Ende des potentiodynamischen Zyklus, der zur Aufzeichnung des Untergrundstromes (der Grundstrom ist nach unten verschoben worden; er wird durch die unterbrochene Linie dargestellt) dient, zugegeben. An der Eichkurve (Figur 3) können die zu den Strommaxima gehörenden Konzentrationen abgelesen werden. Der Umsatz wird in Abhängigkeit von der Zeit (maximal 30 Minuten) bestimmt. Figur 5 stellt die Hydrolyse von D,L-BAPADA mit 5,5 µg/ml (Kurve 1), 8,3 µg/ml (Kurve 2) und 11,0 µg/ml Trypsin (Kurve 3) in Abhängigkeit von der Zeit dar. Die Bedingungen sind dieselben wie diejenigen, die für die Aufzeichnung der maximalen anodischen Stromwerte beschrieben worden sind. Figur 6 stellt den Umsatz in Abhängigkeit von der Konzentration des Enzyms dar (Kurve 1 nach 5 Minuten und Kurve 2 nach 20 Minuten Hydrolyse). Es ist eine Reaktion erster Ordnung. Die klassischen Konstanten wie Km und Vm werden auf dem üblichen Weg aus einem LINEWEAVER-BURK-Diagramm, das die spezifische Reaktionsgeschwindigkeit in Abhängigkeit von der Konzentration des Substrates darstellt, bestimmt.

Figur 7 stellt ein derartiges LINEWEAVER-BURK-Diagramm für die Hydrolyse von D,L-BAPADA mit Trypsin (10 µg/ml) dar. Als Ordinate ist der reziproke Wert der Geschwindigkeit (1/V) und als Abszisse derjenige der Konzentration des Substrates (1/S) aufgetragen worden. Der reziproke Wert der Geschwindigkeit 1/V wurde wie folgt ausgedrückt: entweder als $(\mu A/min - 10\ \mu g\ Enzym)^{-1}$ oder als (µ Mol — $10^3$ pADA/min — 10 µg Enzym). Die Punkte O stellen einen Mittelwert von 10 gemessenen Geschwindigkeitswerten und die Punkte X einen graphischen Mittelwert dar. Jeder Punkt wurde unter Einsatz einer neuen Enzymstammlösung gewonnen.

Experimentelle Bedingungen:

| | |
|---|---|
| Trypsinkonzentration | 10 µg/ml |
| Temperatur | 30° C |
| pH | 8,15 (TRIS) |
| DMSO | 6,35% |

Die MICHAELIS-MENTEN-Konstante Km und die maximale Geschwindigkeit Vm, die aus dem in Figur 7 dargestellten LINEWEAVER-BURK-Diagramm erhalten werden können, betragen $8,10^{-4}$ Mole/Liter und $9,6 - 10^{-10}$ Mole/min µg.

VIII. Messung der Enzymaktivität von Thrombin

Die Verfahrensweise ist ähnlich wie mit Trypsin und ist auch zur Bestimmung weiterer Enzyme, auch mit Hilfe anderer Substrate geeignet. In Tabelle 1 sind die Daten zusammengefaßt. Für Thrombin sind die kinetischen Daten: $Km = 10^{-5}$ Mole/Liter und $Vm = 1,1 \cdot 10^{-7}$ Mole/min × I.E.

| Enzym | Trypsin | Thrombin |
|---|---|---|
| Substrat | Benzoyl-D,L-Arginin-p-Aminodiphenyl-Amid, HCl (D,L-BAPADA) | H-D-Phenylalanyl-L-Pipecolyl-L-Valyl-L-Arginyl-p-Aminodiphenylamid, 2 HCl (HDL) |
| Freigesetzte elektroaktive Spezies | p-Aminodiphenylamin (pADA) | pADA |
| Substratlösung S | 7,65 mg D,L-BAPADA in 1 ml DMSO | 0,40 mg in 1 ml Veronal®-Puffer |
| Versuchslösung A | 0,2 ml der Lösung S + 3 ml 0,025 M Veronal®-Puffer pH 8,15 + NaCl (Ionenstärke 0,15) | 0,4 ml der Lösung S + 2 ml Veronalpuffer |
| Standardisierung | Nach der Hydrolyse des Substrates mit 1 mM Trypsin während einer Minute ergibt sich ein Strom von 0,295 $\mu A/cm^2$ Elektrodenoberfläche | Die Hydrolyse von 0,1 mM Substrat mit 0,01 I. E. Thrombin während einer Minute ergibt einen Strom von 0,217 $\mu A/cm^2$ |
| Ergebnisse | Verhältnis zwischen der Enzymkonzentration E (in µg/ml) und der Hydrolysegeschwindigkeit zur Zeit 0 (durch das Verhältnis (di/dt)o dargestellt) mit einer Elektrode mit 1 $cm^2$ Fläche E = 3,39 (di/dt)o E = in µg/ml (di/dt)o in µA/min. | Verhältnis zwischen der Enzymkonzentration E (in I. E.) und der Hydrolysegeschwindigkeit zur Zeit 0 (durch das Verhältnis (di/dt)o dargestellt) mit einer Elektrode von 1 cm Fläche E = 0,046 (di/dt)o (Dimensionen: s. links) |

## IX. Antithrombin in Blut

Das Blut wird wie üblich auf Natriumzitrat versetzt. Es werden 0,2 ml dieses Blutes in 2,8 ml Veronal®-Puffer mit 4000 I.E./Liter Heparin eine Minute lang bei 40°C inkubiert. Dann werden 50 µl einer Thrombinlösung mit 60 I.E./ml hinzugefügt und 4 Minuten inkubiert. Es werden dann unter Rühren 250 µl einer auf 40°C vorinkubierten Lösung von HDL, wozu 1 ml einer 0,7 mg HDL enthaltenden Lösung mit 2 mg Veronal®-Puffer verdünnt worden waren, hinzugefügt. Nach genau 30 Sekunden Inkubation wird die Reaktion durch Zugabe von Eisessigsäure blockiert und der Strom gemessen. Aus den Meßwerten einer Probe T mit Thrombin und Puffer aber ohne Blut und einer Probe P mit Thrombin, Puffer und Blut und unter der Annahme, daß die für normales Blut gemessenen Werte Tn und Pn sind, ergibt sich die Aktivität des Antithrombins nach der Formel

$$\frac{i_T - i_P}{i_{Tn} - i_{Pn}} \cdot 100$$

in % der Antithrombinaktivität bezogen auf normales Blut.

## X. Herstellung des Substrates D,L-BAPADA

Es werden 4,68 g (0,0168 Mol) Benzoyl-L-Arginin in einer Mischung von einem Äquivalent 85%iger Orthophosphorsäure und 25 ml Diethylphosphit gelöst und in einem Ölbad erhitzt. Dazu werden 1 Äquivalent pADA, 2 Äquivalente reines Triethylamin und eine Lösung von 2 Äquivalenten Phsophorpentaoxyd in 20 ml Diethylphosphit hinzugefügt. Das Ganze wird geschüttelt und die Reaktionsmischung unter gutem Rühren und unter Stickstoff erhitzt. Es wird dann abgekühlt und das Rohprodukt von der Mutterlauge abgetrennt. Es werden dann 300 ml 1 n-HCl zugefügt, und es wird erwärmt. Über Nacht wird im Kühlschrank aufbewahrt und die Oberphase abdekantiert. Das verbleibende Öl wird mit eiskaltem Azeton extrahiert und gewaschen. Aus einer Äther/Äthanol-Mischung wird dann rekristallisiert.

Die Ausbeute beträgt etwa 55%.

Elementaranalyse: (BAPADA = $C_{25}H_{29}O_2N_6Cl$)

| | C | H | N | O | P | Cl |
|---|---|---|---|---|---|---|
| Berechnet: | 62,5 | 6,02 | 17,5 | 6,67 | 0 | 7,38 |
| Gefunden: | 60,9 | 6,16 | 16,6 | 8,60 | 0,2 | 7,20 |

Die IR-, UV- und NMR-Spektren zeigen, daß das erhaltene Produkt wirklich D,L-BAPADA ist.

XI. Kinetische Konstanten von Trypsin und Thrombin

Diese (nachstehend in Tabelle II zusammengefaßten) Konstanten sind mit Hilfe der entsprechend dem Beispiel I f) vorbereiteten Elektrode erzielt worden. Die benutzten Puffer sind die folgenden:

a: Tris (0,025 M), NaCl (0,12 M), Glycin (0,05 M), DMSO 4,76%
Temperatur: 20° C, pH = 8,3
b: Veronal® (0,025 M), NaCl (0,12 M), DMSO 6,35%
Temperatur: 30° C, pH = 8,4

Tabelle II

| Enzym | Substrat | $V_M$ | $K_M$ |
|---|---|---|---|
| Trypsin | Benzol-D,L-Arginin-pADA-Hydrochlorid | 0,00096 | $8 \cdot 10^{-4}$ (b) |
| Thrombin | H-D-Phe-Pip-Arg-pADA | 0,10 | $1 \cdot 10^{-5}$ (a) |
| | H-D-Phe-Pip-Arg-4-MBNA | 0,020 | $3{,}7 \cdot 10^{-5}$ (a) |

pADA: Para-Aminodiphenylamid
MBNA: Methoxy-$\beta$-Naphthylamid
$V_M$: in µMol/min I. E. ausgedrückt Maximalgeschwindigkeit
$K_M$: Michaelis-Konstante in Mol/l

XII. Bestimmung des Enzymhydrolyseproduktes in nichtwäßrigem Medium

Die Arbeitsweise entspricht derjenigen des Beispieles IX, wobei jedoch 2 ml Chloroform hinzugefügt wurden, nachdem die Hydrolyse durch Ansäuern mit einem gleichen Volumen 50%iger Essigsäure oder 0,15 M Salzsäure blockiert worden war. Nach heftigem Schütteln wird die wäßrige Oberphase abgesaugt. Falls eine Emulsion vorliegt, kann durch etwa 10minütiges Einlegen von Molekularsiebkugeln in die Lösung eine homogene Phase erhalten werden. Der auf diese Weise gewonnenen organischen Phase werden 2 ml Dimethylsulfoxyd und quarternäres Ammoniumperchlorat hinzugefügt, um das Medium leitend zu machen. Die Menge des vorhandenen Hydrolyseproduktes wird dann durch Voltametrie ermittelt. Die Korrelation zwischen dem Oxydationsstrom und der Konzentration des Hydrolyseproduktes ergibt sich aus einer Eichkurve, die an einer Lösung erhalten wurde, deren Zusammensetzung identisch war mit derjenigen der Probe.

Wie aus dem Text hervorgeht, ist die Erfindung nicht nur auf die beschriebenen Verfahren, Ausführungsformen und Anwendungen beschränkt, sondern umfaßt auch alle Varianten, die den Fachmann beim Lesen dieser Beschreibung ohne weiteres einfallen.

## Patentansprüche

1. Verfahren zur Bestimmung einer Protease oder Antiprotease, dadurch gekennzeichnet, daß man in einem wäßrigen Medium das Enzym mit einem Substrat A–B zusammenbringt, worin A einen Peptidrest, dessen terminales Stickstoffatom gegebenenfalls substituiert und dessen C-terminale Aminosäure Arginin ist, oder N-substituiertes Arginyl, und B den Rest eines Amins H–B bedeutet, das elektrochemisch oxydiert oder reduziert werden kann, und das abgespaltene Amin mittels Amperometrie bestimmt.

2. Verfahren nach Anspruch 1, dadurch gekennzeichnet, daß man als Enzym eine Protease oder

Antiprotease des Blutgerinnungssystems oder des Komplements mit dem Substrat zusammenbringt.

3. Verfahren nach Anspruch 1, dadurch gekennzeichnet, daß die Amperometrie mittels eines Gerätes mit einem Potentiostat und einer Meßzelle mit zwei oder drei Elektroden, einer Meßelektrode, einer Bezugselektrode und/oder einer Hilfselektrode ausgeführt wird.

4. Verfahren nach einem der Ansprüche 1 oder 3, dadurch gekennzeichnet, daß die Veränderung der Konzentration des elektroaktiven Produktes in der Meßzelle aus dem periodisch im zeitlichen Abstand gemessenen Oxydations- oder Reduktionsstrom bestimmt wird.

5. Verfahren gemäß Anspruch 4, dadurch gekennzeichnet, daß der Oxidations- oder Reduktionsstrom aus dem Peak in der Strom-Spannungs-Charakteristik bestimmt wird, die sich ergibt, wenn ein Potential mit sägezahnförmigem Verlauf angelegt wird.

6. Verfahren nach einem der Ansprüche 1, 3, 4 oder 5, dadurch gekennzeichnet, daß die Meßelektrode einer Vorkonditionierung ausgesetzt wird, die aus einer ununterbrochenen Abtastung mit einem Sägezahnpotential besteht, das sich zwischen −0,1 V und +0,4 V mit einer Geschwindigkeit von 0,2 V/sec ändert, wobei diese Abtastung beendet wird, wenn das Signal $i = f(U)$ eine Grenzkurve erreicht hat.

7. Verfahren gemäß Anspruch 3, dadurch gekennzeichnet, daß die Messung mit einer Elektrode aus einem Edelmetall wie Platin, Gold oder Silber, aus Edelstahl oder aus Kohlenstoffpaste ist, die durch Mischen von Kohlenstoffpulver mit Paraffinöl erhalten wird, als Meßelektrode ausgeführt wird.

8. Verfahren gemäß Anspruch 1, dadurch gekennzeichnet, daß die Bestimmung in einem wäßrigen Medium erfolgt.

9. Verfahren entsprechend Anspruch 1, dadurch gekennzeichnet, daß die Bestimmung in einem nicht wäßrigen Medium erfolgt.

10. Verfahren gemäß Anspruch 1, dadurch gekennzeichnet, daß das Produkt vor der Bestimmung abgetrennt wird.

11. Verfahren gemäß Anspruch 1, dadurch gekennzeichnet, daß als Substrat eine substituierte Aminosäure oder ein Oligopeptid, die als Produkt ein aromatisches oder heterozyklisches Amin oder Polyamin freisetzen, verwendet wird.

12. Verfahren gemäß Anspruch 11, dadurch gekennzeichnet, daß als Substrat ein Substrat verwendet wird, das durch Substitution mit einer Acyl-, Sulfonyl-, Tosyl- oder Benzoyl-Gruppe aktiviert ist.

13. Verfahren gemäß Anspruch 11, dadurch gekennzeichnet, daß als Amin p-Amino-diphenylamin, 4,4'-Benzidin oder o-Anisidin verwendet wird.

14. Verfahren gemäß Anspruch 11, dadurch gekennzeichnet, daß als Amin 4-Methoxy-2-Naphthylamin oder p-Nitranilin verwendet wird.

15. Verfahren nach Anspruch 11, dadurch gekennzeichnet, daß als substituierte Aminosäure- oder der Oliogopeptidrest Benzoyl-Arginyl, Tosyl-Arginyl, Tosyl-L-Glycyl-L-Prolyl-L-Arginyl, H-D-Phenylalanyl-L-Pipecolyl-L-Arginyl, N-Benzolyl-L-Phenylalanyl-L-Valyl-L-Arginyl oder H-D-Phenylalanyl-L-Pipecolyl-L-Arginyl verwendet wird.

16. Verfahren gemäß Anspruch 8, dadurch gekennzeichnet, daß das Reaktionsmedium 2 bis 10% Dimethylsulfoxyd enthält.

**Claims**

1. Process for the determination of a protease or antiprotease, which comprises bringing together, in an aqueous medium, the enzyme and a substrate A—B in which A denotes a peptide radical, the terminal nitrogen atom of which is optionally substituted and the C-terminal aminoacid of which is arginine, or N-substituted arginyl, and B denotes the radical of an amine H—B which can be oxidized or reduced electrochemically, and determining the amine which has been split off, by means of amperometry.

2. Process as claimed in claim 1, wherein the enzyme brought together with the substrate is a protease or antiprotease of the blood clotting system or of the complement.

3. Process as claimed in claim 1, wherein amperometry is carried out by means of equipment which has a potentiostat and a measurement cell with two or three electrodes, a measurement electrode, a reference electrode and/or an auxiliary electrode.

4. Process as claimed in one of claims 1 or 3, wherein the change in the concentration of the electroactive product in the measurement cell is determined from the oxidation or reduction current measured periodically at intervals.

5. Process as claimed in claim 4, wherein the oxidation or reduction current is determined from the peak in the current/voltage characteristic which is obtained when a potential with a saw-tooth profile is applied.

6. Process as claimed in one of claims 1, 3, 4 or 5, wherein the measurement electrode has been subjected to a preconditioning which comprises unbroken scanning with a saw-tooth potential which changes between −0.1 V and +0.4 V at a speed of 0.2 V/second, this scanning being terminated when the signal $i = f(U)$ has reached a limiting curve.

7. Process as claimed in claim 3, wherein the measurement is carried out using, as the measurement

electrode, an electrode made of a noble metal, such as platinum, gold or silver, of special steel or of carbon paste, which is obtained by mixing carbon powder with paraffin oil.

8. Process as claimed in claim 1, wherein the determination is carried out in an aqueous medium.

9. Process as claimed in claim 1, wherein the determination is carried out in a non-aqueous medium.

10. Process as claimed in claim 1, wherein the product is separated off prior to the determination.

11. Process as claimed in claim 1, wherein the substrate used is a substituted aminoacid or an oligopeptide which liberates, as the product, an aromatic or heterocyclic amine or polyamine.

12. Process as claimed in claim 11, wherein the substrate used is a substrate which has been activated by substitution with an acyl, sulfonyl, tosyl or benzoyl group.

13. Process as claimed in claim 11, wherein the amine used is p-amino-diphenylamine, 4,4'-benzidine or o-anisidine.

14. Process as claimed in claim 11, wherein the amine used is 4-methoxy-2-naphthylamine or p-nitroaniline.

15. Process as claimed in claim 11, wherein the substituted aminoacid radical or the oligopeptide radical used is benzoyl-arginyl, tosyl-arginyl, tosyl-L-glycyl-L-prolyl-L-arginyl, H-D-phenylalanyl-L-pipecolyl-L-arginyl, N-benzoyl-L-phenylalanyl-L-valyl-L-arginyl or H-D-phenylalanyl-L-pipecolyl-L-arginyl.

16. Process as claimed in claim 8, wherein the reaction medium contains 2 to 10% of dimethylsulfoxide.

**Revendications**

1. Procédé de dosage d'une protéase ou d'une antiprotéase, caractérisé en ce que l'on fait réagir l'enzyme dans un milieu aqueux, sur un substrat de formule A—B, où A représente un radical peptide, dont l'atome terminal d'azote est éventuellement substitué et dont l'aminoacide C-terminal est de l'arginine ou de l'arginyle N-substitué et B représente le reste d'une amine H—B qui peut être réduite ou oxydée par voie électrochimique et en ce que l'on détermine l'amine séparée par ampérométrie.

2. Procédé selon la revendication 1, caractérisé en ce que l'enzyme que réagit sur le substrat A—B est une protéase ou une antiprotéase du système de coagulation sanguine ou du complément.

3. Procédé selon la revendication 1, caractérisé en ce que l'on effectue l'ampérométrie à l'aide d'un appareil comprenant un potentiostat et une cellule de mesure munie de deux ou trois électrodes, une électrode de mesure, une électrode de référence et/ou une électrode auxiliaire.

4. Procédé selon la revendication 1 ou 3, caractérisé en ce que l'on détermine la modification de la concentration du produit électroactif dans la cellule de mesure à partir du courant d'oxydation ou de réduction mesuré périodiquement par intervalles.

5. Procédé selon la revendication 4, caractérisé en ce que le courant d'oxydation ou de réduction est déterminé par l'amplitude du pic ou de la vague voltamétrique obtenu lors du pilotage d'un signal potentiodynamique.

6. Procédé selon l'une quelconque des Revendications 1 à 4, caractérisé en ce que, avant de procéder au dosage proprement dit, l'électrode indicatrice est soumise à un préconditionnement consistant en un balayage ininterrompu de signaux potentiodynamiques triangulaires entre −0,1 V et +0,4 V, à la vitesse de 0,2 V/sec., ledig balayage étant arrêté lorsque l'enregistrement du signal-réponse sur un enregistreur (signal i/v) fait apparaître une courbe-limite.

7. Procédé selon la revendication 3, caractérisé en ce que l'on effectue la mesure à l'aide d'une électrode de mesure en métal noble tel que le platine, l'or ou l'argent, en acier spécial ou en pâte de carbone, préparé en mélangeant de la poudre de carbone avec de l'huile de paraffine.

8. Procédé selon la revendication 1, caractérisé en ce que le dosage est effectué en milieu aqueux.

9. Procédé selon la revendication 1, caractérisé en ce que le dosage est effectué en milieu non aqueux.

10. Procédé selon la revendication 1, caractérisé en ce que le produit est séparé avant le dosage.

11. Procédé selon la revendication 1, caractérisé en ce que, on utilise comme substrat un aminoacide ou oligopeptide substitué qui libère comme produit une amine ou polyamine aromatique ou hétérocyclique.

12. Procédé selon la revendication 11, caractérisé en ce que, on utilise un substrat que est activé par substitution d'un groupe acyle, sulfonyle, tosyle ou benzoyle.

13. Procédé selon la revendication 11, caractérisé en ce que, on utilise comme amine la p-amino-diphénylamine, la 4,4'-benzidine ou la o-anisidine.

14. Procédé selon la revendication 11, caractérisé en ce que, comme amine, on utilise la 4-méthoxy-2-naphthylamine ou la p-nitraniline.

15. Procédé selon la revendication 1, caractérisé en ce que, comme reste d'aminoacide ou d'oligopeptide substitué, on utilise le benzoyle-arginyle, le tosyle-arginyle, le tosyle-L-glycyl-L-prolyl-L-arginyle, le H-D-phénylalanyl-L-pipecolyl-L-arginyle, le N-benzolyl-L-phénylalanyl-L-valyl-L-arginyle ou le H-D-phénylalanyl-L-pipecolyl-L-arginyle.

16. Procédé selon la revendication 8, caractérisé en ce que le milieu réactionnel contient de 2 à 10% de diméthylsulfoxyde.

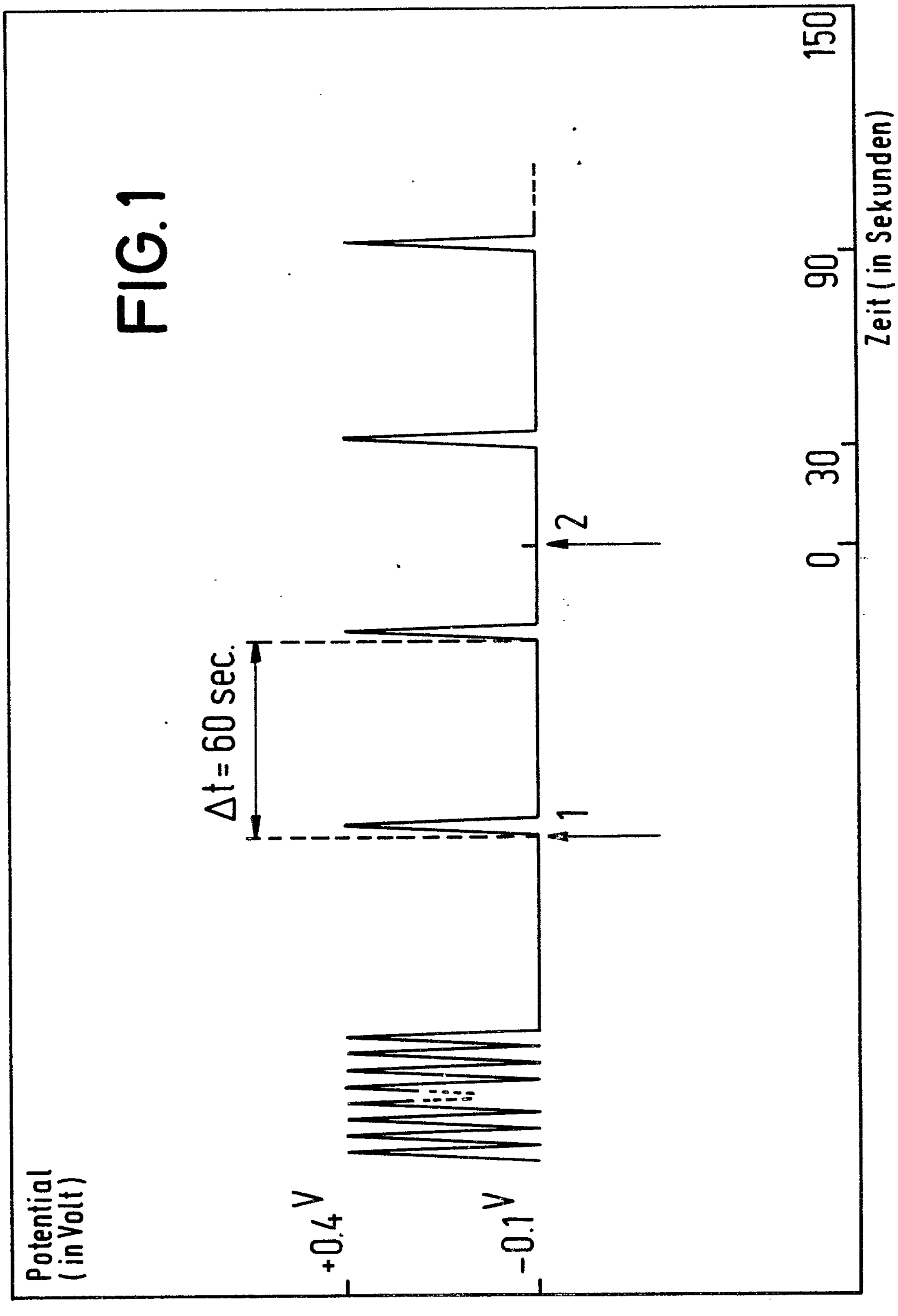
Potential
( in Volt )
+0,4 V
−0,1 V
Δt = 60 sec.
1
2
0
30
90
150
Zeit ( in Sekunden )

FIG. 1

FIG. 2

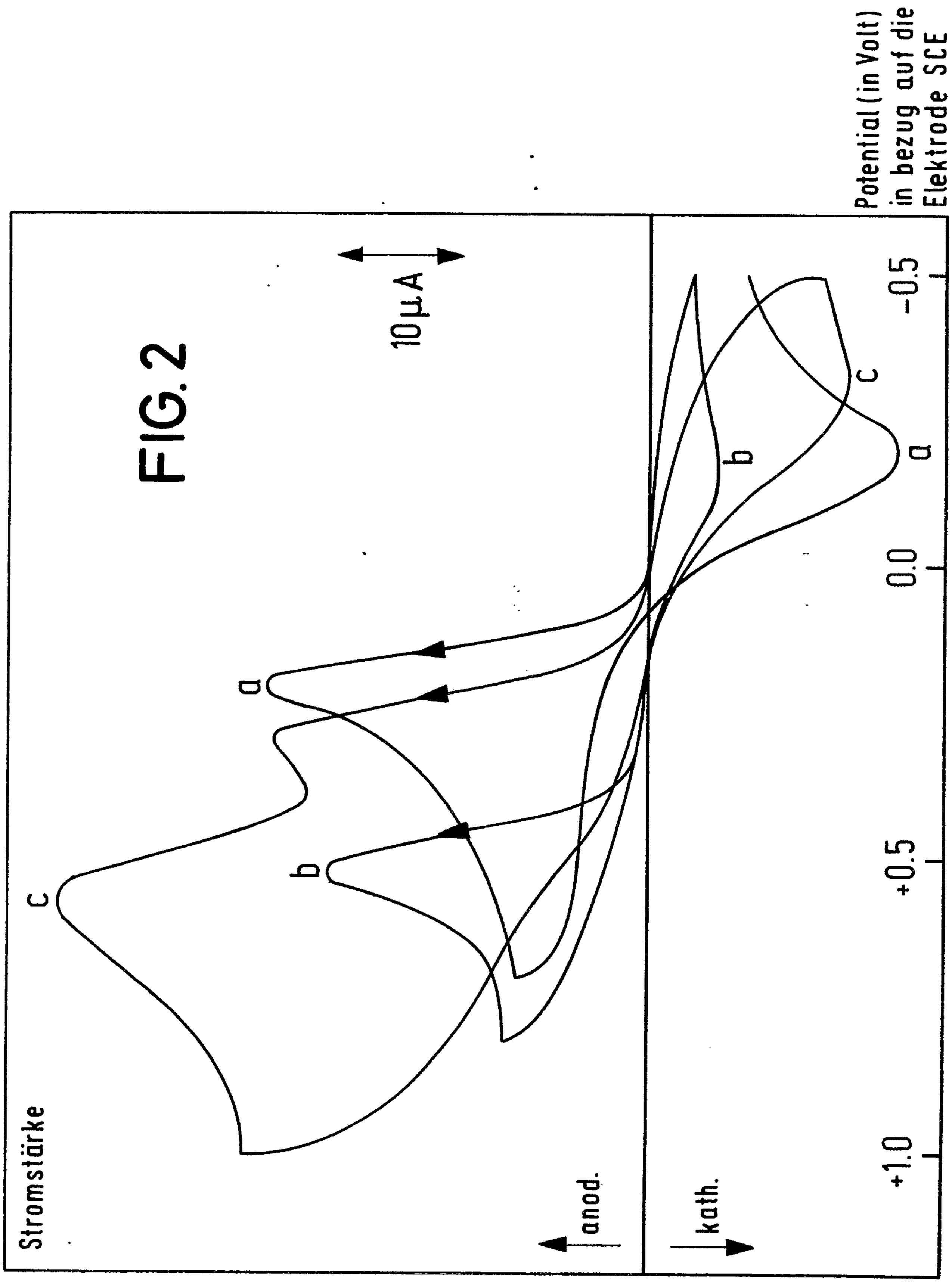
Stromstärke
anod.
kath.
10 µA
a
b
c
+1.0
+0.5
0.0
−0.5
Potential (in Volt)
in bezug auf die
Elektrode SCE

FIG. 3

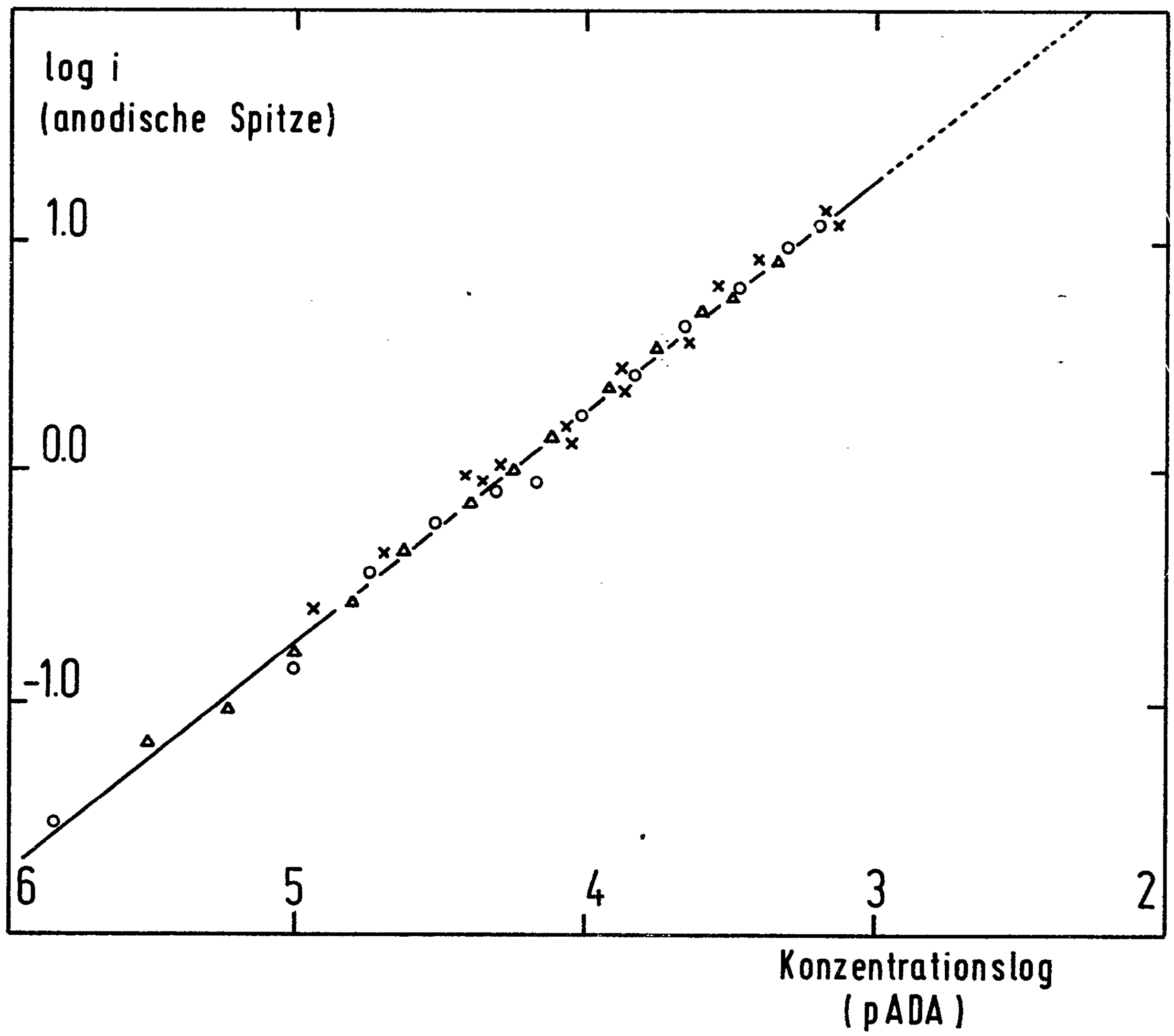

log i
(anodische Spitze)
1.0
0.0
-1.0
6
5
4
3
2
Konzentrationslog
(pADA)

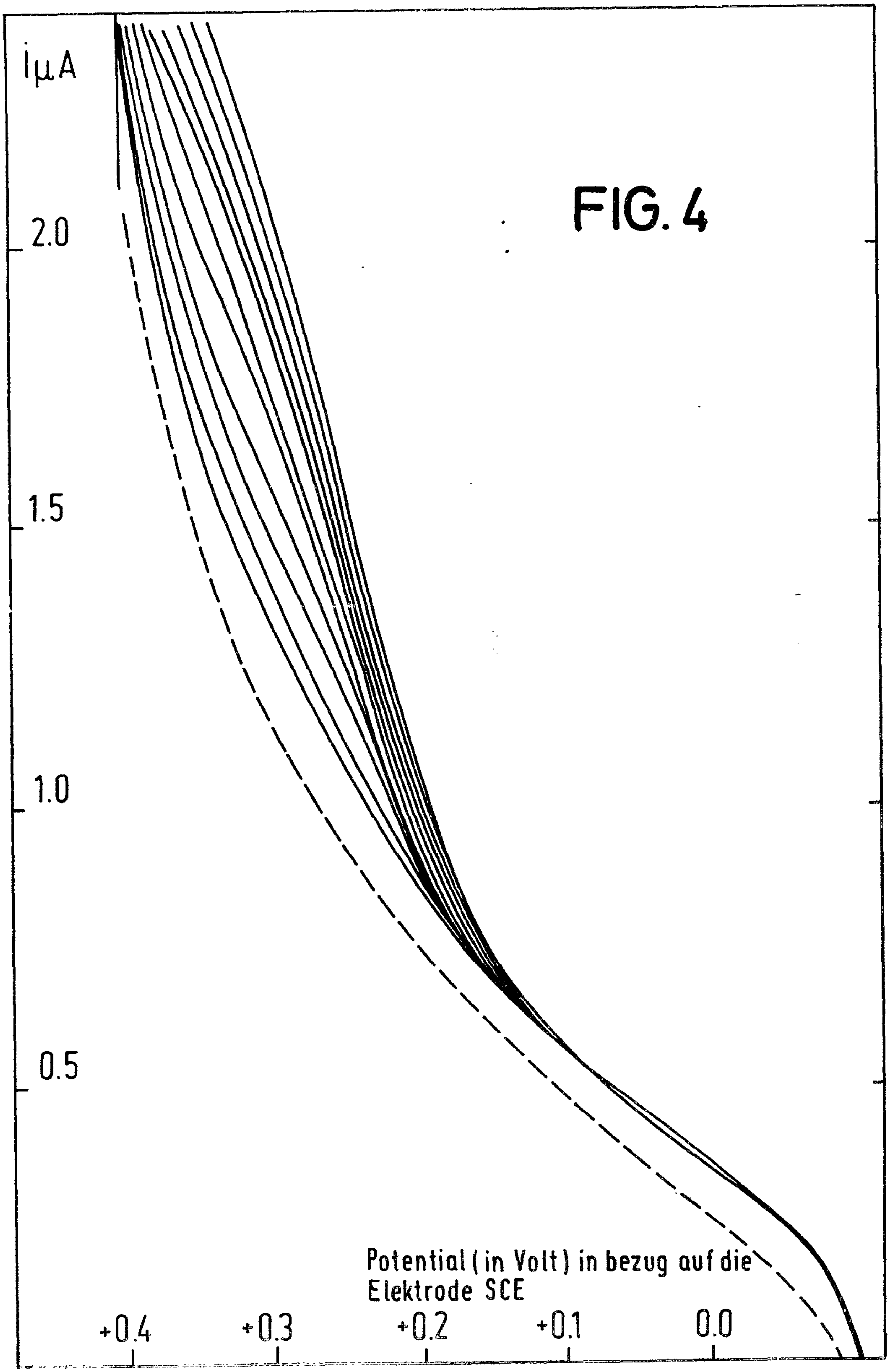
i µA
2.0
1.5
1.0
0.5
Potential (in Volt) in bezug auf die Elektrode SCE
+0.4
+0.3
+0.2
+0.1
0.0

FIG. 4

FIG. 5

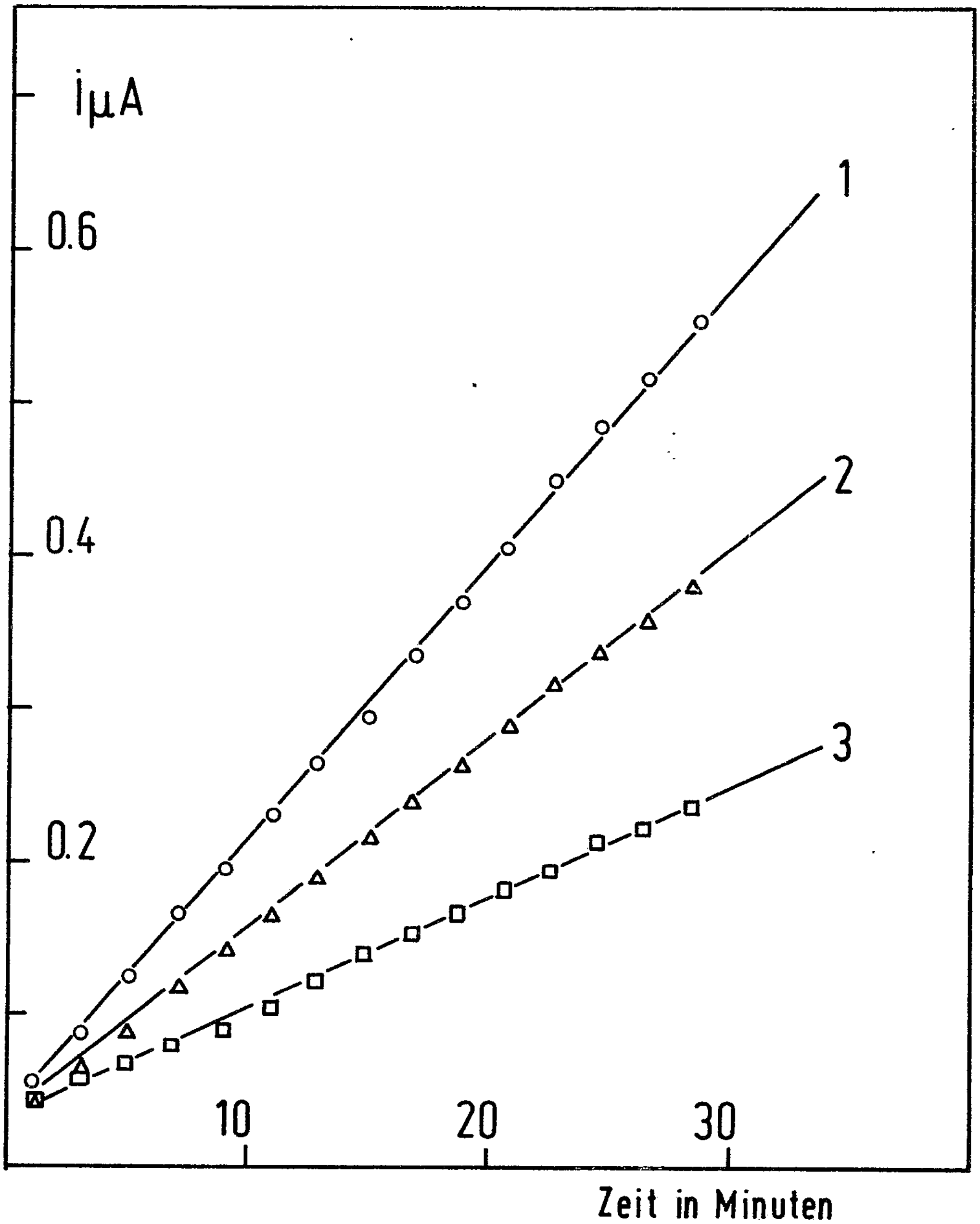

FIG. 6

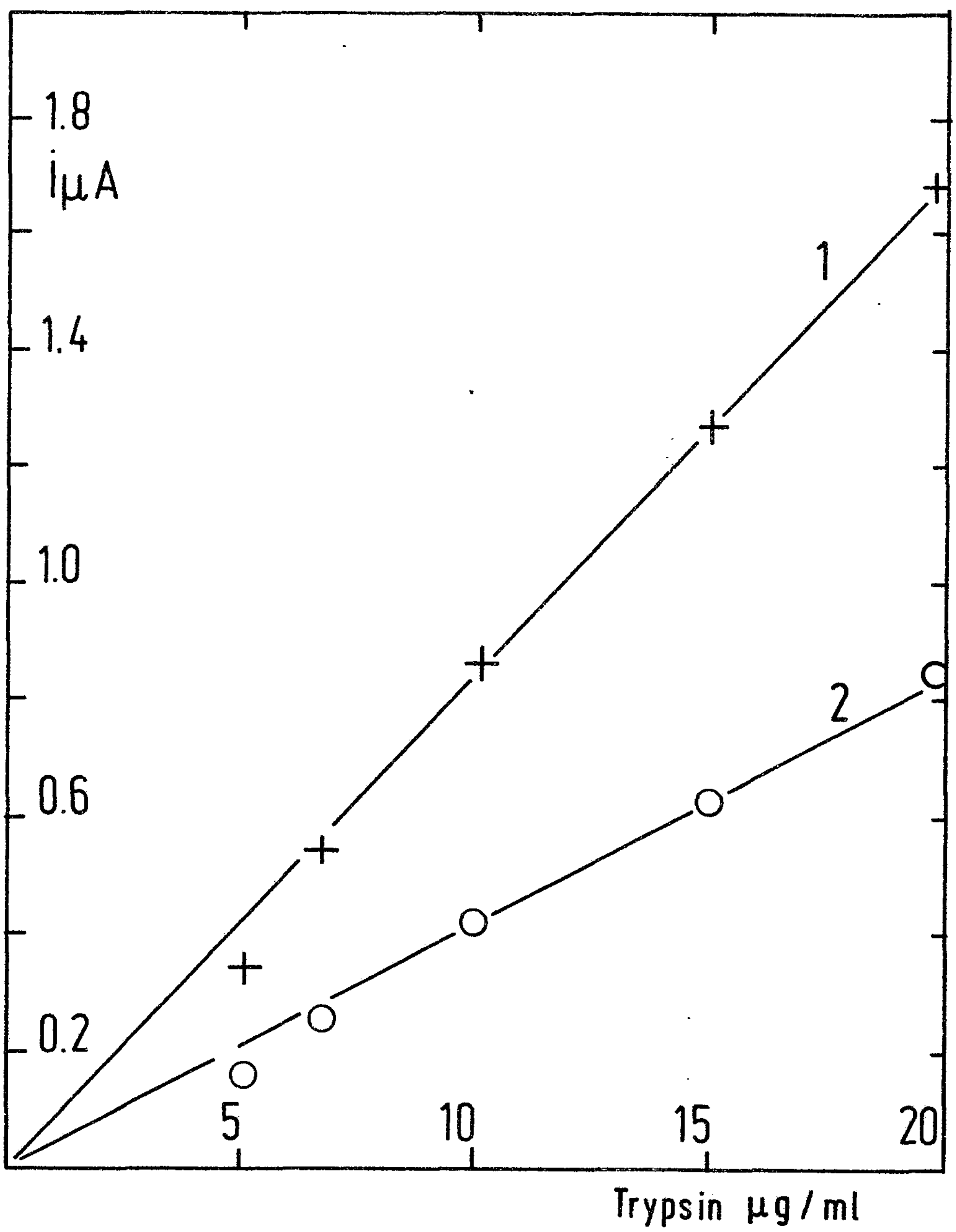

FIG. 7

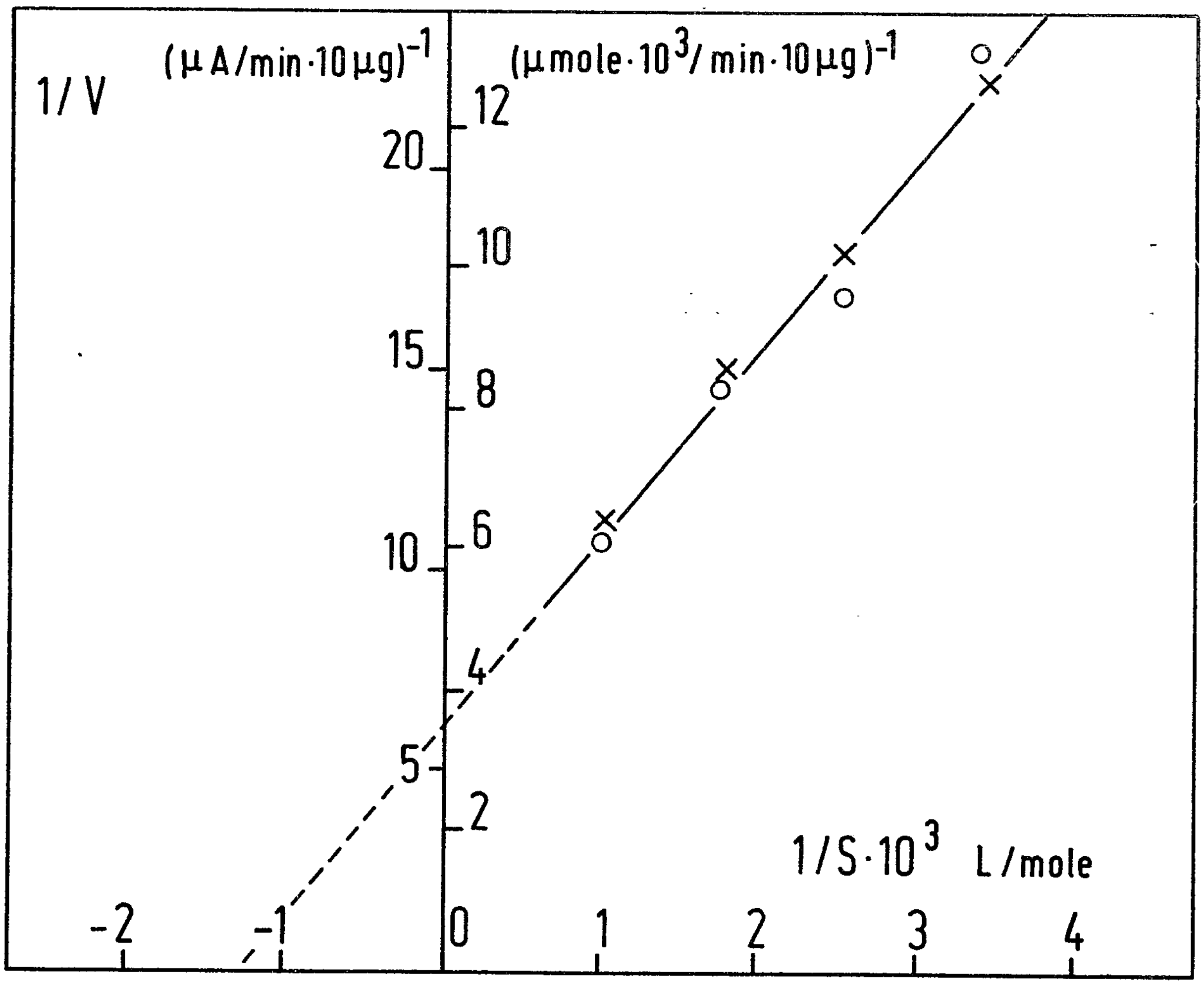

1/V
(μA/min·10μg)-1
(μmole·10³/min·10μg)-1
20
15
10
5
12
10
8
6
4
2
1/S·10³ L/mole
-2
-1
0
1
2
3
4

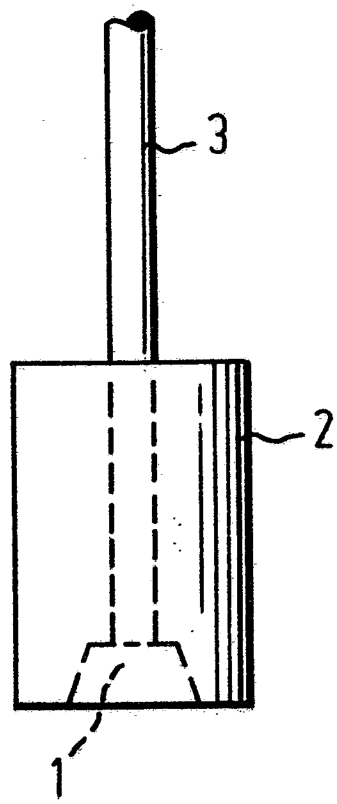


FIG.8

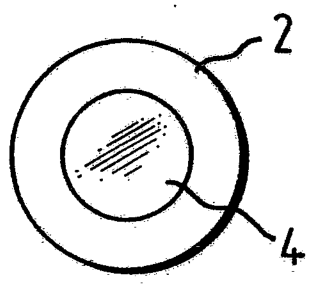


FIG. 9